# EUROPEAN PATENT SPECIFICATION

(11) **EP 3 646 834 B1**
(45) Date of publication and mention of the grant of the patent: **10.08.2022**
(21) Application number: 18848306.9
(22) Date of filing: 26.02.2018
(51) Int. Cl.: A61F 13/494, A61F 13/49, A61F 13/496, A61F 5/44, A61F 13/531

(54) **ABSORBENT ARTICLE**
ABSORBIERENDER ARTIKEL
ARTICLE ABSORBANT

(30) Priority: 23.08.2017 JP 2017160118
(43) Date of publication of application: 06.05.2020
(73) Proprietor: Unicharm Corporation, Shikokuchuo-shi, Ehime 799-0111 (JP)
(72) Inventor: HASHIMOTO, Tatsuya, Kanonji-shi Kagawa 769-1602 (JP); KATSURAGAWA, Kunihiko, Kanonji-shi Kagawa 769-1602 (JP); MATSUSHIMA, Yuta, Kanonji-shi Kagawa 769-1602 (JP)
(74) Representative: Dolleymores
(86) International application number: PCT/JP2018/006967
(87) International publication number: WO 2019/038956

(56) References cited:
- EP-A1- 3 009 115
- EP-A1- 3 162 332
- JP-A- H 053 891
- JP-A- 2009 061 046
- JP-A- 2014 104 351
- JP-A- 2015 188 501
- JP-A- 2015 213 543

## Description

### [Technical Field]

The present invention relates to an absorbent article.

### [Background Art]

Absorbent articles such as pull-on disposable diapers are conventionally known. There is desire for a reduction in the cost of absorbent articles, and one method of lowering the cost of an absorbent article is a method of reducing the basis weight of liquid-absorbent fibers (e.g., pulp fibers) of the absorbent body (absorbent core), for example. As one example, PTL 1 discloses a technique of providing a constricted portion 5c in an absorbent core 5 of a pull-on disposable diaper 1 in order to reduce the size of the aggregate of fibers such as pulp fibers in the absorbent core 5, for example.

### [Citation List]

### [Patent Literature]

[PTL 1] Japanese Patent Application Publication No. 2013-13683

EP 3009115 A1 relates to a disposable pull-on diaper including an absorbent chassis and a lower torso cover which assumes a tubular shape and defines a front waist region, a back waist region and a crotch region. The absorbent chassis extends between the front waist region and the back waist region and has a front end portion overlapping the front waist region and a back end portion overlapping the back waist region. The lower torso cover is provided with an elastic region.

### [Summary of Invention]

### [Technical Problem]

However, with the pull-on disposable diaper 1 in PTL 1, there are cases where excrement cannot be sufficiently absorbed by the absorbent core 5 that has a reduced amount of liquid-absorbent fibers, and there is a risk that excrement leaks from the pull-on disposable diaper 1.

The present invention was achieved in light of conventional problems such as that described above, and an aspect of the present invention is to reduce the amount of liquid-absorbent fibers or the like of the absorbent body while also further reducing the risk that excrement leaks from the absorbent article.

### [Solution to Problem]

The present invention provides the absorbent article of independent claim 1. The dependent claims specify preferred but optional features.

Features of the present invention other than the above will become clear by reading the description of the present specification with reference to the accompanying drawings.

### [Advantageous Effects of Invention]

According to this underpants-shaped absorbent article, even when excrement is absorbed by the absorbent body that has the narrow portion in the region where the absorbent main body and the waist portion are overlapped with each other in the thickness direction, it is possible to reduce the risk that excrement leaks out from the absorbent article.

### [Brief Description of the Drawings]

FIG. 1 is a schematic perspective view of a pull-on disposable diaper 1.
FIG. 2 is a schematic plan view of the diaper 1 in an unfolded and stretched state, as viewed from the skin-side surface.
FIG. 3 is a schematic cross-sectional view taken along a line A-A in FIG. 2.
FIG. 4A is a schematic cross-sectional view taken along a line B1-B1 in FIG. 2. FIG. 4B is a schematic cross-sectional view taken along a line B2-B2 in FIG. 2. FIG. 4C is a schematic cross-sectional view taken along a line B3-B3 in FIG. 2.
FIG. 5 is a schematic diagram illustrating the configuration in the schematic plan view of FIG. 2.
FIG. 6A is a diagram illustrating a method of forming an absorbent main body 10 and leg portions 60. FIG. 6B is a schematic cross-sectional view taken along a line D-D in FIG. 6A.
FIG. 7A is an illustrative diagram illustrating states before and after excrement is absorbed by the absorbent main body 10 in a region indicated by line B1-B1 in FIG. 2. FIG. 7B is an illustrative diagram illustrating states before and after excrement is absorbed by the absorbent main body 10 in a region indicated by line B2-B2 in FIG. 2.

### [Description of Embodiments]

At least the following matters will become clear with the description of this specification and the attached drawings.

An absorbent article having a up-down direction, a left-right direction, and a front-back direction that intersect each other,
the absorbent article including:
an absorbent main body including an absorbent body, a core-wrapping sheet having a skin-side sheet portion and a non-skin-side sheet portion, and a pair of leak-proof walls,
   the absorbent body having a superabsorbent polymer,
   the skin-side sheet portion located on a skin side of the absorbent body,
   the non-skin-side sheet portion located on a non-skin side of the absorbent body,
   the pair of leak-proof walls each having a leak-proof-wall elastic member;
a front waist portion; and
a back waist portion,
the absorbent body including a narrow portion that has a left-right-direction side end having a recessed portion,
the leak-proof walls each including a fixed portion and a rising portion,
   the fixed portion being not capable of rising to the skin side,
   the rising portion being capable of rising to the skin side,
out of the front waist portion and the back waist portion,
   at least one-side waist portion that is located on one side being overlapped with a non-skin side of the narrow portion,
   the at least one-side waist portion being overlapped in the up-down direction with at least a portion of the narrow portion,
an adhesive being applied to at least either one of a non-skin-side surface of the skin-side sheet portion and a skin-side surface of the non-skin-side sheet portion,
   the application being made in a region that is outside the narrow portion and above a lower end of the one-side waist portion,
at least a portion of the fixed portion being overlapped with the skin side of the absorbent body,
when an intersection between the lower end of the one-side waist portion and the side end of the narrow portion is used as a reference,
   a rise start portion of the rising portion being located outward in the left-right direction with respect to the intersection.

According to this underpants-shaped absorbent article, even when excrement is absorbed by the absorbent body that has the narrow portion in the region where the absorbent main body and the waist portion are overlapped with each other in the thickness direction, it is possible to reduce the risk that excrement leaks out from the absorbent article.

In such an absorbent article, it is desirable
that the one-side waist portion has a waist elastic member that extends in the left-right direction, and
that at least a portion of the waist elastic member straddles the side end of the narrow portion in the left-right direction.

According to this absorbent article, it is possible to suppress the risk that the relatively stiff absorbent main body folds so as to protrude to the non-skin side in the approximately left-right-direction central portion, and make it more likely for the absorbent main body to fit to the wearer's body.

In such an absorbent article, it is desirable that
at least a portion of the waist elastic member intersects the leak-proof-wall elastic member.

According to this absorbent article, it is possible to further suppress the risk that the relatively stiff absorbent main body folds so as to protrude to the non-skin side in the approximately left-right-direction central portion, and make it more likely for the absorbent main body to fit to the wearer's body.

In such an absorbent article, it is desirable
that the one-side waist portion has a waist elastic member that extends in the left-right direction, and
that in a stretched state where the waist elastic member and the leak-proof-wall elastic member are stretched,
   concerning an upper recession end that is an upper end of a located-on-the-one-side and recessed portion of the side end,
   the waist elastic member that is located below the upper recession end is not provided inward in the left-right direction with respect to the upper recession ends when the upper recession end is used as a reference.

According to this absorbent article, spaces (hereinafter, also called "leak-prevention spaces") are defined by the rising portions on the left-right-direction outward side and by the narrow portion on the left-right-direction inward side, and are defined by the rising portions on the skin side and by the one-side waist portion on the non-skin side, and it is possible to suppress the risk that these spaces collapse and can no longer hold excrement.

In the absorbent article,
in a stretched state where the leak-proof-wall elastic member is stretched,
concerning an upper recession end that is an upper end of a located-on-the-one-side and recessed portion of the side end,
an up-down-direction length from the upper recession end to the lower end of the one-side waist portion
is longer than
a left-right-direction length from the upper recession end to an intersection between the lower end of the one-side waist portion and the side ends.

Accordingly, it is possible to suppress the risk that excrement stored in the leak-prevention space leaks toward the wearer's crotch.

In such an absorbent article, it is desirable that
in a stretched state where the leak-proof-wall elastic member is stretched,
concerning an upper recession end that is an upper end of a located-on-the-one-side and recessed portion of the side end,
a portion of the side end is inclined with respect to the up-down direction, the portion of the side end being located between the upper recession end and the lower end of the one-side waist portion.

According to this absorbent article, in the leak-prevention space, the length of the side end of the absorbent body is longer than in the case where the side end of the absorbent body are parallel with the up-down direction. This makes it possible to increase the area of contact between excrement and the absorbent body, and thus more easily absorb excrement.

In such an absorbent article, it is desirable that
on the one side, an upper end of the narrow portion is located below a lower end of the fixed portion.

According to this absorbent article, the size of the leak-prevention spaces can increase when the absorbent body expands due to absorbing excrement.

In such an absorbent article, it is desirable
that in a stretched state where the leak-proof-wall elastic member is stretched,
an inner end of each of the leak-proof wall is located inward in the left-right direction with respect to the intersection.

According to this absorbent article, the leak-proof wall covers the absorbent body up to a location that is outward in the left-right direction with respect to the ends of the absorbent body. This makes it possible to suppress the risk that excrement absorbed by the absorbent body leaks out from the absorbent article.

In such an absorbent article, it is desirable that
in a stretched state where the leak-proof-wall elastic member is stretched,
the leak-proof walls each have an adhesion portion for preventing rising of the fixed portion to the skin side, and
the adhesion portion is located inward with respect to the side ends of the absorbent body.

According to this absorbent article, the leak-prevention spaces can be more easily formed when the absorbent body expands due to absorbing excrement.

In such an absorbent article, it is desirable that
in a stretched state where the leak-proof-wall elastic member is stretched,
the leak-proof walls each have an adhesion portion for preventing rising of the fixed portion to the skin side, and
the area of the adhesion portions is greater than the area of the fixed portions minus the area of the adhesion.

According to this absorbent article, compared to the case where the area of the adhesion portions is smaller than the area of the fixed portions minus the area of the adhesion portions, the leak-prevention spaces can be more easily formed when the absorbent body expands due to absorbing excrement.

In such an absorbent article, it is desirable
that the one-side waist portion has a plurality of waist elastic members that extend in the left-right direction, and
that in a stretched state where the waist elastic members and the leak-proof-wall elastic member are stretched,
   concerning a first waist elastic member that is one of the plurality of waist elastic members and that is located lowest in the one-side waist portion, and
   concerning a second waist elastic member that is one of the plurality of waist elastic members and that is vertically adjacent to the first waist elastic member,
      a length from the lower end of the one-side waist portion to the first waist elastic member is longer than a length between the first waist elastic member and the second waist elastic member.

According to this absorbent article, the length from the lower end of the one-side waist portion to the first waist elastic member is longer than the length from the first waist elastic member to the second waist elastic member, and therefore excrement can more easily enter the leak-prevention space. On the other hand, a shorter distance between the first waist elastic member and the second waist elastic member also makes it possible to further suppress the risk that excrement in the leak-prevention space leaks out.

In such an absorbent article, it is desirable that
in a stretched state where the leak-proof-wall elastic member is stretched,
the leak-proof-wall elastic member includes a first leak-proof-wall elastic member and a second leak-proof-wall elastic member in the rising portions, and
the first leak-proof-wall elastic member and the second leak-proof-wall elastic member are separated from each other.

According to this absorbent article, the multiple leak-proof-wall elastic members that are provided at separated positions makes it more likely for the leak-proof walls to be in surface-to-surface contact with the wearer's skin when the leak-proof-wall elastic members are in the risen state, thus making it possible to improve the skin texture.

### Embodiments

The following describes embodiments of an absorbent article according to the present invention by way of example of a "pull-on disposable diaper". The pull-on disposable diaper of the present embodiment can be used for infants or adults. Also, besides a disposable diaper, the underpants-shaped absorbent article according to the present invention can be a shorts-type sanitary napkin, for example.

### Basic configuration of pull-on disposable diaper

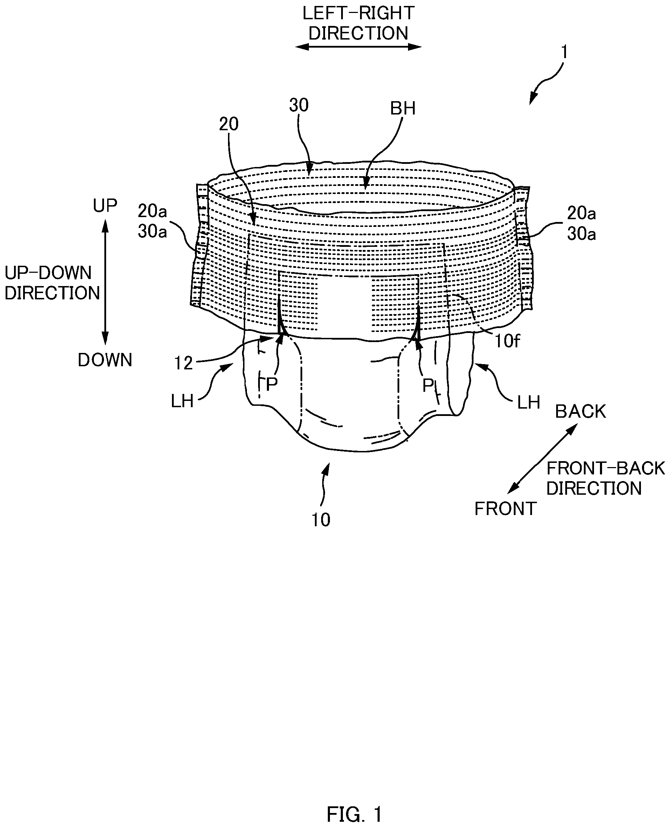

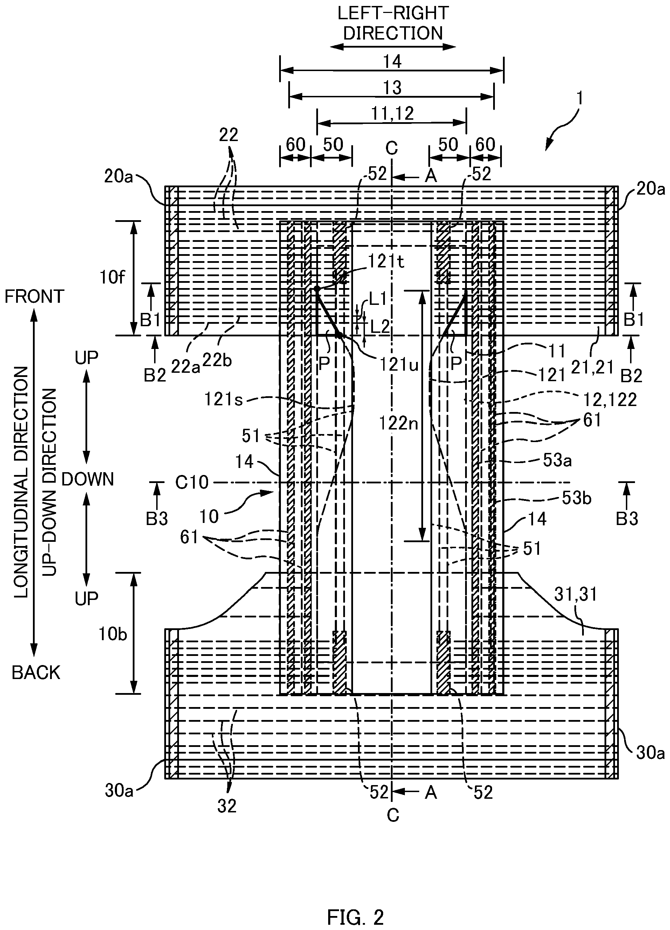

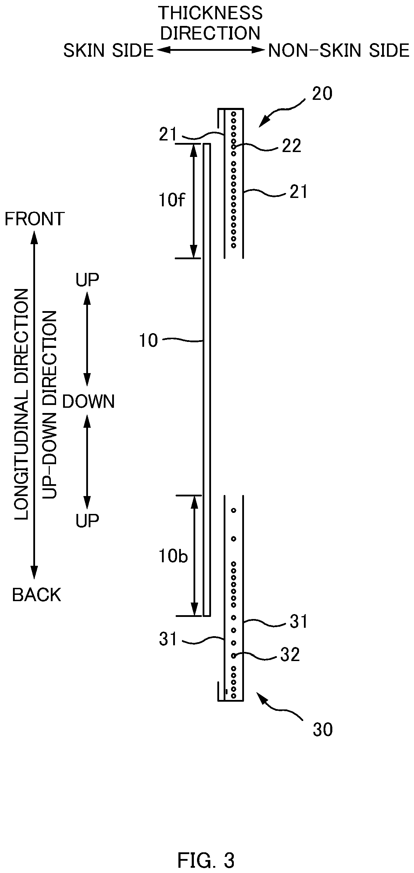

FIG. 1 is a schematic perspective view of a pull-on disposable diaper 1 (hereinafter, also called the "diaper 1"). FIG. 2 is a schematic plan view of the diaper 1 in an unfolded and stretched state, as viewed from the skin-side surface. FIG. 3 is a schematic cross-sectional view taken along a line A-A in FIG. 2. The "unfolded state" is a state where side portions 20a of a front waist portion (also called the "stomach-side waist portion") 20 and side portions 30a of a back waist portion 30 (also called the "back-side waist portion") on the two sides of the diaper 1 are detached from each other and opened to lay the whole diaper 1 flat. The "stretched state" is a state where elastic members provided in the diaper 1 are stretched until wrinkles can no longer be seen in the diaper 1. Specifically, this is a state in which the diaper 1 is stretched until the dimensions of constituent members thereof (e.g., the later-described front waist portion 20) match or are close to the dimensions of such members on their own. A line C-C in FIG. 2 is the center line in the left-right direction. Adhesive is not shown in FIG. 3 for the sake of convenience.

As shown in FIG. 1, the pull-on diaper 1 has an up-down direction, a left-right direction, and a front-back direction, and the diaper 1 includes a waist opening BH and a pair of leg openings LH. In the up-down direction, the waist opening BH side is the upper side, and the side corresponding to the wearer's crotch is the lower side. The up-down direction of the diaper 1 in the state in FIG. 2 (unfolded and stretched state) will also be called the "longitudinal direction", one side and the other side in the longitudinal direction will also be respectively called the "front side" and the "back side", and the side corresponding to an approximately central portion C10 side in the longitudinal direction will also be called the "lower side". In the front-back direction, the side corresponding to the wearer's stomach is the front side, and the side corresponding to the wearer's back is the back side. The diaper 1 also has a thickness direction as shown in FIG. 3, and the side that comes into contact with the wearer in the thickness direction is the skin side, and the side opposite thereto is the non-skin side.

The diaper 1 is a so-called three-piece type of diaper, and has an absorbent main body 10, a front waist portion 20, and a back waist portion 30. The front waist portion 20 covers the wearer's stomach region, and the back waist portion 30 covers the wearer's back region. The absorbent main body 10 is approximately rectangular in a plan view. As shown in FIG. 3, a front upper end portion 10f and a back upper end portion 10b of the absorbent main body 10 are respectively overlaid on the skin-side surfaces of the waist portions 20 and 30. Note that the front upper end portion 10f is the front end portion of the absorbent main body 10, and is the portion that is overlapped with the waist portion 20. The back upper end portion 10b is the back-side end portion of the absorbent main body 10, and is the portion that is overlapped with the back waist portion 30. The front waist portion 20 and the back waist portion 30 are approximately rectangular in a plan view, and the longitudinal directions thereof conform to the left-right direction.

As shown in the unfolded state in FIG. 2, the shape of the diaper 1 has left-right symmetry about the center line C-C. The non-skin-side surfaces of the front upper end portion 10f and the back upper end portion 10b of the absorbent main body 10 are respectively joined to the skin-side surfaces of the waist portions 20 and 30 with use of an adhesive or the like (not shown) . Further, the absorbent main body 10 in the unfolded state shown in FIG. 2 is then folded one time such that the front waist portion 20 and the back waist portion 30 oppose each other, and two left-right-direction side portions 20a of the front waist portion 20 are joined to two left-right-direction side portions 30a of the back waist portion 30, thus obtaining the pull-on diaper 1.

The front waist portion 20 and the back waist portion 30 each include two soft sheets (21 and 21, 31 and 31) made of nonwoven fabric or the like, and respectively include multiple waist elastic members 22 and 32 (elastic strings or the like) that stretch and contract in the left-right direction. The waist elastic members 22 and 32 are arranged side-by-side with gaps therebetween in the up-down direction, and are fixed between the two corresponding sheets (21 and 21, 31 and 31) while being stretched in the left-right direction. The front waist portion 20 and the back waist portion 30 can therefore stretch and contract in the left-right direction, and fit to the wearer's waist.

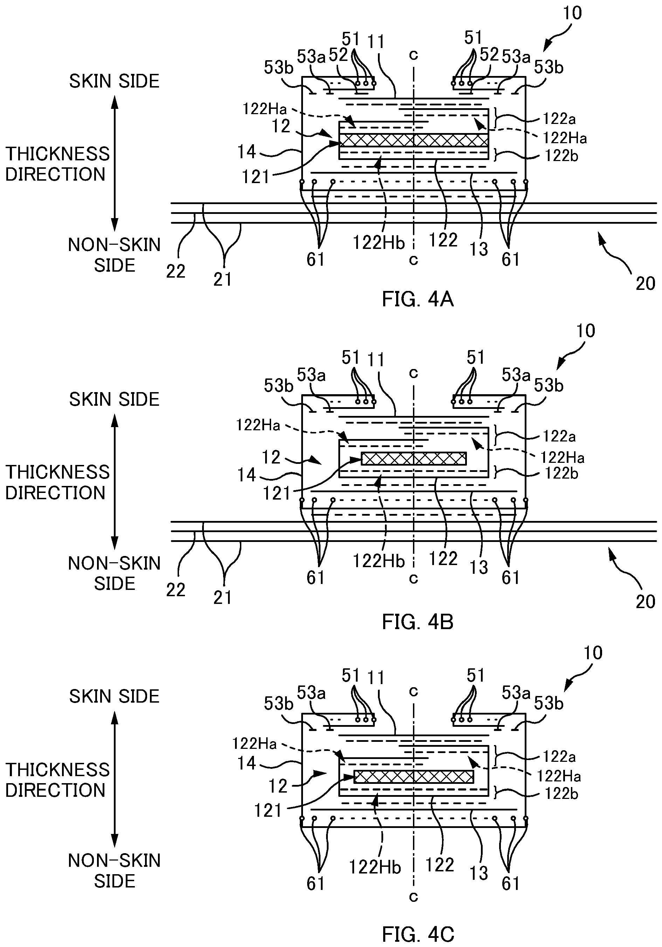

FIG. 4A is a schematic cross-sectional view taken along a line B1-B1 in FIG. 2. FIG. 4B is a schematic cross-sectional view taken along a line B2-B2 in FIG. 2. FIG. 4C is a schematic cross-sectional view taken along a line B3-B3 in FIG. 2. In FIGS. 4A, 4B, and 4C, the dimensions of members have been changed as necessary in order to facilitate understanding of the configuration with respect to the thickness direction. As shown in FIGS. 4A, 4B, and 4C, the absorbent main body 10 includes a top sheet 11, an absorbent body unit 12, a back sheet 13, and an exterior sheet 14 in this order from the skin side in the thickness direction, and these members are adhered using an adhesive such as a hot-melt adhesive. The top sheet 11 may be any type of liquid-permeable sheet, and examples thereof include a hydrophilic air-through nonwoven fabric sheet and a spunbond nonwoven fabric sheet. The back sheet 13 may be any type of liquid-impermeable sheet, and examples thereof include a polyethylene film sheet, a polypropylene film sheet, and a hydrophobic SMS nonwoven fabric sheet. The top sheet 11 and the back sheet 13 have a size sufficient for covering the entirety of the absorbent body unit 12. The exterior sheet 14 may be a liquid-permeable sheet or a liquid-impermeable sheet, and one example thereof is a hydrophobic SMS nonwoven fabric sheet. It should be noted that leak-proof wall portions 50 are formed by the exterior sheet 14 in the present embodiment, and therefore it is preferable that the exterior sheet 14 is made of a liquid-impermeable material.

The absorbent body unit 12 is approximately rectangular in a plan view, and includes an absorbent core (also simply called the "absorbent body") 121 that absorbs liquids, and a core-wrapping sheet 122 that covers the outer peripheral surfaces of the absorbent core 121. As one example, the liquid absorbent material is made of liquid-absorbent fibers (e.g., pulp fibers) that contain a superabsorbent polymer (so-called SAP) . The core-wrapping sheet 122 is, for example, a liquid-permeable sheet made of tissue paper, nonwoven fabric, or the like.

The core-wrapping sheet 122 has skin-side adhesive portions 122Ha that are provided on a skin-side sheet portion 122a that is located on the skin side of the absorbent core 121, and non-skin-side adhesive portions 122Hb that are provided on a non-skin-side sheet portion 122b that is located on the non-skin side of the absorbent core 121. In the present embodiment, the skin-side adhesive portions 122Ha are provided over approximately the entirety of the skin-side sheet portion 122a, and the non-skin-side adhesive portions 122Hb are provided over approximately the entirety of the non-skin-side sheet portions 122b. However, it is not necessary that these adhesive portions Ha and Hb are provided over the entirety of the sheet portions 122a and 122b respectively. A configuration is possible in which at least either the skin-side sheet portion 122a or the non-skin-side sheet portion 122b is provided with the adhesive portions 122Ha or 122Hb. Also, in the present embodiment, a configuration is possible in which the skin-side adhesive portions 122Ha and the non-skin-side adhesive portions 122Hb are provided at least in a region that is above the lower end of the front waist portion 20 and outside a narrow portion 121n (described later). In other words, a configuration is possible in which the adhesive portions 122Ha or 122Hb are provided at least outward of the absorbent core 121 which includes the narrow portion 121n, in a region upward of the lower end of the front waist portion 20, as shown in FIG. 4B. Note that it is preferable that the skin-side sheet portion 122a and the non-skin-side sheet portion 122b are adhered to the absorbent core 121 to the extent that it is possible to mitigate the risk that separation of the sheet portions 122a and 122b from the absorbent core 121 cause a poor fit when the diaper is worn or cause a loss of the predetermined shape of the absorbent core 121.

The absorbent core 121 is obtained by shaping the liquid absorbent material into a predetermined shape. Specifically, as shown in FIG. 2, the longitudinal central portion of the absorbent core 121 is narrowed so as to have a shorter length in the left-right direction, thus forming a narrow portion 121n whose left-right-direction side ends 121s are recessed, and therefore the absorbent core 121 is approximately hourglass-shaped. The narrow portion 121n is formed so as to be more located on the front side than on the back side, and a narrowest portion 121m of the narrow portion 121n is located above the approximately longitudinal central portion C10. Upper recession ends 121t of the side ends 121s of the absorbent core 121, which are the upper ends of the recessed portions, are arranged at locations that are above the lower end of the front waist portion 20 and below the lower ends of end joining portions 52 (described later) . Also, intersections 121u between the lower end of the front waist portion 20 and the side ends 121s of the absorbent core 121 are located inward in the left-right direction with respect to the upper recession ends 121t.

The non-skin-side surface of the narrow portion 121n is overlaid on the skin-side surface of the front waist portion 20, and the front waist portion 20 will also be called the "one-side waist portion". Also, the narrow portion 121n is not overlaid on the back waist portion 30.

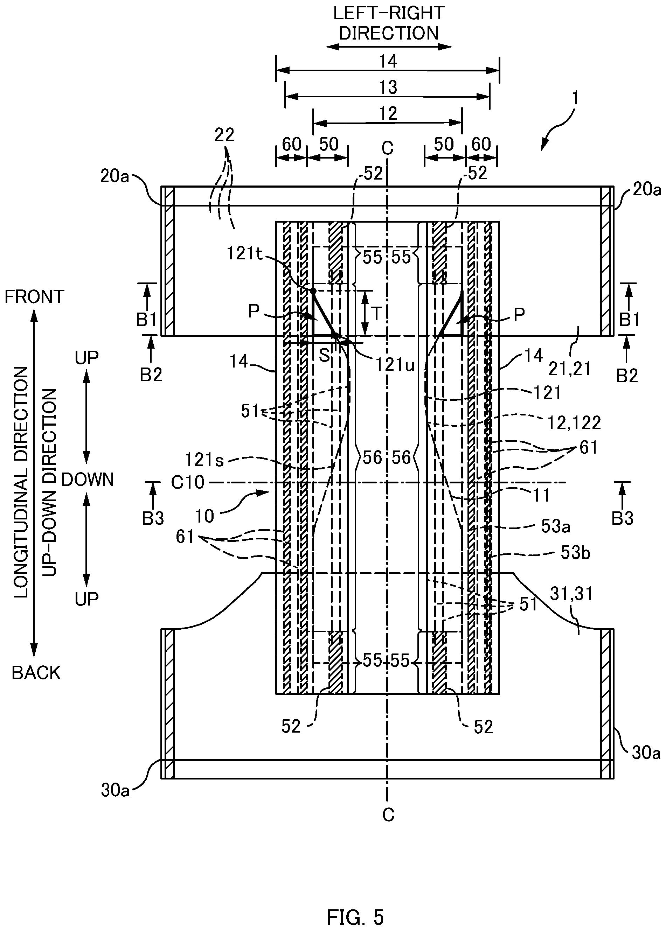

The absorbent main body 10 also includes a pair of leak-proof wall portions 50 and leg portions 60. Although described in detail later, in the present embodiment, as shown in FIGS. 4A to 4C, 5A and the like, the two left-right-direction side portions of the exterior sheet 14 are folded back, and in the folded-back portions of the exterior sheet 14, the left-right-direction inner end portions form the leak-proof wall portions (also called "leak-proof walls") 50, and the left-right-direction outer end portions form the leg portions 60.

The pair of leak-proof wall portions 50 are so-called barrier cuffs, and are located on the two left-right-direction side portions of the absorbent body unit 12, with extending along the longitudinal direction of the absorbent main body 10. The leak-proof wall portions 50 each include: a pair of end joining portions 52 that join the exterior sheet 14, which forms the leak-proof wall portion 50, to the top sheet 11 and the back sheet 13; multiple leak-proof-wall elastic members 51 that stretch and contract in the longitudinal direction (up-down direction of the diaper 1); fixed portions 55 that cannot rise to the skin side; and rising portions 56 that can rise to the skin side. Also, the leg portions 60 each include side joining portions 53 and leg elastic members 61 that stretch and contract in the longitudinal direction.

FIG. 5 is a schematic diagram illustrating the configuration in the schematic plan view of FIG. 2. FIG. 5 shows a view in which the waist elastic members 22 and 32 and the like have been omitted from the schematic plan view in FIG. 2. The pair of end joining portions 52 and the pair of side joining portions 53 are portions that have been joined using an adhesive such as a hot-melt adhesive. Note that the joining of the end joining portions 52 and the side joining portions 53 is not limited to fixing with use of adhesive, and such fixing may be performed through compression, pressure bonding, or the like.

The pair of end joining portions 52 are arranged at the two longitudinal end portions of the absorbent main body 10, and are "adhesion portions" for preventing the fixed portions 55 from rising to the skin side. The end joining portions 52 extend in the longitudinal direction from the end of the absorbent main body 10 to positions overlapped with the upper end portion of the absorbent body unit 12.

The side joining portions 53 extend from one longitudinal end of the absorbent main body 10 to the other longitudinal end and are provided in pairs on both sides outward in the left-right direction with respect to the end joining portions 52. Each side joining portion includes a side joining portion 53a located on the inside in the left-right direction and a side joining portion 53b located on the outside in the left-right direction. The leak-proof wall portions 50 can rise, due to contraction of the leak-proof-wall elastic members 51, toward the wearer (to the skin side in the thickness direction) from portions that are inward in the left-right direction and adjacent to the side joining portions 53a. The leak-proof wall portions 50 each have a fixed portion 55 that extends from the upper end of the front waist portion 20 to the lower end of the end joining portion 52 on that side, and a fixed portion 55 that extends from the upper end of the back waist portion 30 to the lower end of the end joining portion 52 on that side. In other words, the rising portion 56 is the region of the leak-proof wall portion 50 that is below the lower end of the end joining portion 52 on the front waist portion 20 side and above the lower end of the end joining portion 52 on the back waist portion 30 side. The leak-proof wall portions 50 stop the lateral flow of excrement. Note that the leak-proof wall portions 50 can be formed by one leak-proof-wall elastic member 51, but it is preferable that multiple leak-proof-wall elastic members 51 are arranged separated from each other as in the diaper 1. Using multiple leak-proof-wall elastic members 51 facilitates realizing surface-to-surface contact between the leak-proof wall portions 50 and the wearer's skin when the leak-proof wall portions 50 are in the risen state, thus making it possible to improve the skin texture.

Note that although string-like elastic members such as elastic strings are illustrated as the elastic members (22, 32, 51 and 61) provided in the front waist portion 20, the back waist portion 30, the leak-proof wall portions 50, and the leg portions, there is no limitation to this. Instead of elastic strings, it is possible to use one or more sheet-like elastic members made of a stretchable film, stretchable nonwoven fabric, or the like. In the drawings, shown are only portions of the elastic members that exhibit stretchability (so-called effective length portions). Therefore outside the illustrated portions of the elastic member in the longitudinal direction, the elastic members may have portions that do not exhibit stretchability. Also, the number of and arrangement of the elastic members are also not limited to the configuration shown in the drawings.

### Method of forming leak-proof wall portions 50 and leg portions 60

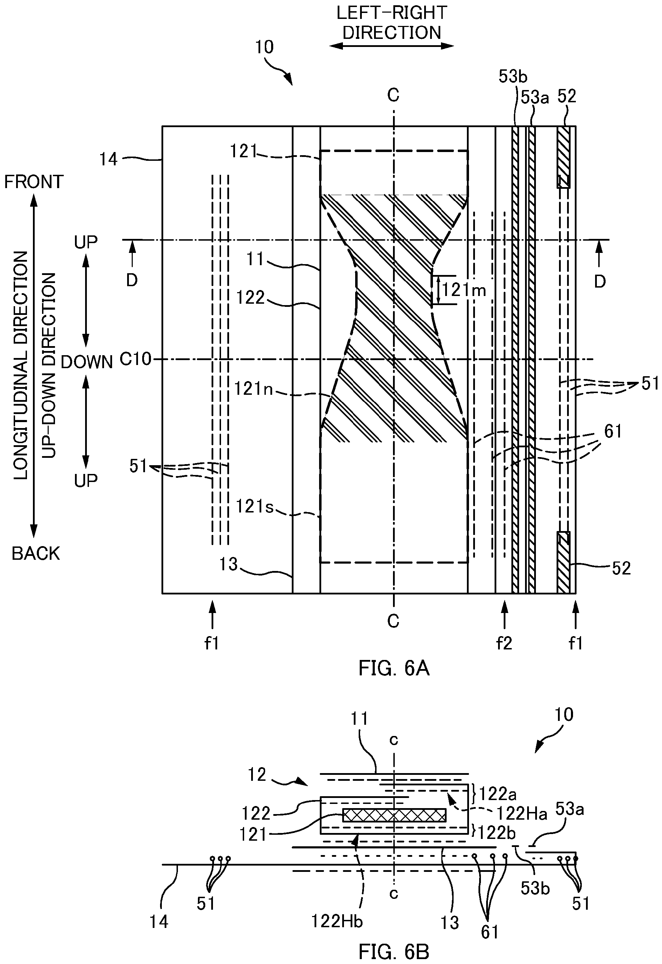

The following briefly describes a method of forming the pair of leak-proof wall portions 50 of the absorbent main body 10 with reference to FIGS. 6A and 6B. FIG. 6A is a diagram illustrating a method of forming the absorbent main body 10 and the leg portion 60. FIG. 6B is a schematic cross-sectional view taken along a line D-D in FIG. 6A.

In FIG. 6A, the narrow portion 121n region of the absorbent core 121 is indicated by groups of three lines extending diagonally downward and rightward. A portion 121m of the narrow portion 121n that has the shortest length in the left-right direction is located on the front side of the approximately longitudinal central portion C10.

First, as shown on the left side in FIGS. 6A and 6B, three leak-proof-wall elastic members 51 for the leak-proof wall portion 50 are fixed in each of two left-right-direction inward side portions of the exterior sheet 14, in a state of being stretched in the longitudinal direction. Thereafter, the two left-right-direction side portions of the exterior sheet 14 are folded to the skin side in the thickness direction (inward in the left-right direction) at folding positions f1 that are the positions in the left-right direction where the leak-proof-wall elastic members 51 for the leak-proof wall portions 50 were fixed.

Next, as shown on the right side in FIGS. 6A and 6B, adhesive for forming the end joining portions 52 and the side joining portions 53 of each leak-proof wall portion 50 is applied to portions that are on the skin-side surface of the two-layer portions of the exterior sheet 14 and are outward in the left-right direction with respect to a folding position f2. Then, three leg elastic members 61 are fixed to portions that are on the skin-side surface of the exterior sheet 14 and are inward in the left-right direction with respect to the side joining portions 53, in a state of being stretched in the longitudinal direction. Thereafter, the back sheet 13 and the absorbent body unit 12 having the top sheet 11 arranged thereon are overlaid and joined onto the left-right-direction central portion of the exterior sheet 14.

Lastly, at the folding positions f2 that are between the absorbent body unit 12 and the side joining portions 53b that are located on the inside in the left-right direction, the two left-right-direction side portions of the exterior sheet 14 are folded toward the skin side in the thickness direction with respect to the top sheet 11, thus obtaining the absorbent main body 10 shown in FIG. 2 and the like. The leg portions 60 are formed by regions of the folded exterior sheet 14 that are outside the inner side ends of the left-right-direction-inside, side joining portions 53a. And, the leak-proof wall portions 50 are formed by the regions that are inside the inner ends of the side joining portions 53a.

### Aspects of absorbent main body 10 when excrement has been absorbed

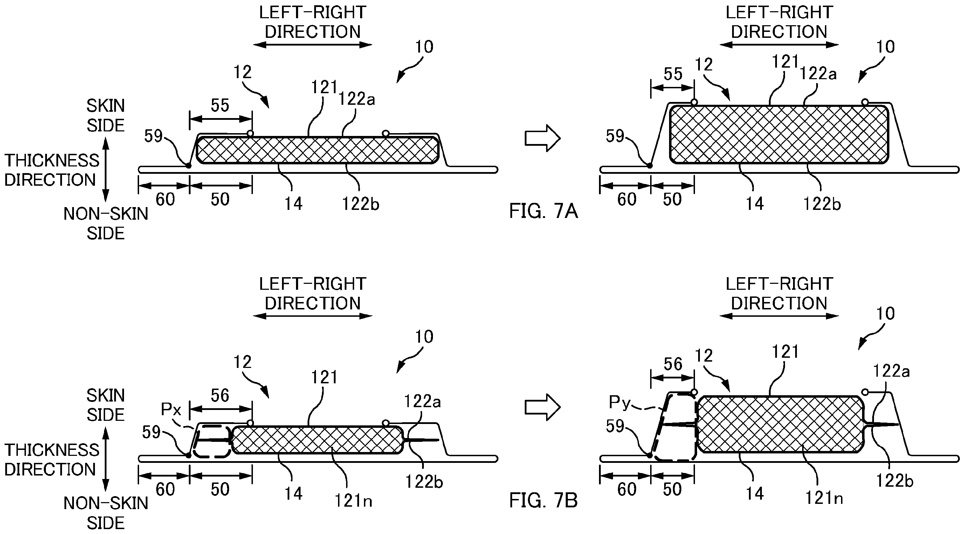

FIG. 7A is an illustrative diagram illustrating states before and after excrement is absorbed by the absorbent main body 10 in a region indicated by line B1-B1 in FIG. 2. FIG. 7B is an illustrative diagram illustrating states before and after excrement is absorbed by the absorbent main body 10 in a region indicated by line B2-B2 in FIG. 2. FIG. 7A shows a cross-section of the absorbent main body 10 at the lower ends of the end joining portions 52 (fixed portions 55), and FIG. 7B shows a cross-section of the absorbent main body 10 at the lower end of the front waist portion 20. In FIGS. 7A and 7B, the left side shows the absorbent main body 10 before excrement has been absorbed, and the right side shows the absorbent main body 10 after excrement has been absorbed. Note that FIGS. 7A and 7B are illustrative diagrams, and the dimensions, scale, and the like of members are not necessarily accurate.

Normally, when the wearer (infant or the like) of the diaper 1 excretes excrement while wearing the diaper 1, the excrement is absorbed by the absorbent body unit 12 in the vicinity of the wearer's crotch region. There are cases where the amount of liquid-absorbent fibers (e.g., pulp fibers) of the absorbent core 121 or the like is reduced in order to lower the cost of the diaper 1, and as shown in FIG. 2 and the like, there are cases where the absorbent core 121 is approximately hourglass-shaped and has the narrow portion 121n that has recessed left-right-direction side ends. Forming the narrow portion 121n in the absorbent core 121 has the advantage of lowering the risk that the relatively stiff absorbent core 121 limits movement of the wearer's legs at their crotch.

However, if the amount of liquid-absorbent fibers is reduced, there is a risk that excrement is not completely absorbed by the absorbent core 121 in the crotch portion, and that excrement flows toward the front waist portion 20 or the back waist portion 30. In particular, there has been a risk that fast-flowing excrement flows from portions where the absorbent main body 10 is overlapped with the front waist portion 20 or the back waist portion 30, passes the leak-proof walls, and flows out from the diaper.

In view of this, in the diaper 1 of the present embodiment, the risk of excrement flowing out from the diaper 1 can be reduced by forming a leak-prevention space P shown in FIG. 7B. The leak-prevention space P will be described below.

As shown on the left side in FIGS. 7A and 7B, before absorbing excrement, the absorbent core 121 of the diaper 1 is not in an expanded state due to not having absorbed excrement or the like. At this time, as shown on the left side in FIG. 7A, each leak-proof wall portion 50, which is overlaid on the absorbent body unit 12 in the thickness direction, includes the fixed portion 55 that is provided by the end joining portion 52 (FIG. 5) . In other words, the leak-proof wall portion 50 is fixed to the absorbent body unit 12 in the fixed portion 55. Note that the narrow portion 121n is not provided in the absorbent core 121 in FIG. 7A.

On the other hand, as shown on the left side in FIG. 7B, the leak-proof wall portion 50, which is overlaid on the absorbent body unit 12 in the thickness direction, includes the rising portion 56 that is not fixed by the end joining portion 52 and can rise to the skin side. By rising to the skin side, the leak-proof wall portion 50 forms a wall that stops the flow of excrement. Also, the narrow portion 121n is provided here and has a shorter length in the left-right direction than the absorbent core 121 shown in FIG. 7A. For this reason, in the put-on state or the like, the rising portion 56 rises to the skin side, thus forming a leak-prevention space Px; the leak-prevention space Px is defined in the left-right direction by the rising portion 56 on the outside and by the narrow portion 121n on the inside, and is defined by the rising portion 56 on the skin side and by the front waist portion 20 on the non-skin side. In other words, this leak-prevention space Px is a space that is defined by the leak-proof wall portion 50, the absorbent core 121, and the front waist portion 20. Note that in the diaper 1 shown in FIG. 7B, the exterior sheet 14 is adhered to and integrated with the front waist portion 20, and therefore the leak-prevention space Px, which is defined by the leak-proof wall portion 50, the absorbent core 121, and the exterior sheet 14, can also be said to be defined by the leak-proof wall portion 50, the absorbent core 121, and the front waist portion 20. Note that in FIGS. 1, 2, and 5, solid lines show the positions of the leak-prevention spaces P that are formed when the rising portions 56 have risen.

Also, it is preferable that regions where portions of the core-wrapping sheet 122 are adhered together are provided outward in the left-right direction with respect to the narrow portion 121n. Specifically, it is preferable that the skin-side sheet portion 122a and the non-skin-side sheet portion 122b are adhered together with use of an adhesive. It is not necessarily required that the skin-side sheet portion 122a and the non-skin-side sheet portion 122b are adhered together when the diaper 1 is manufactured. It is sufficient that the skin-side sheet portion 122a and the non-skin-side sheet portion 122b are adhered together when the diaper 1 is put on, and in one example configuration, the skin-side sheet portion 122a and the non-skin-side sheet portion 122b are separate when the diaper 1 is manufactured, and then adhered through pressure when the diaper 1 is placed in a package, or through contact with the wearer's skin when the diaper is put on.

Note that rise start portions 59 refer to the boundaries between the leak-proof wall portions 50 and the leg portions 60, or more specifically, the portions that are inwardly adjacent to the inner ends of the left-right-direction-inside, side joining portions 53a. The leak-proof wall portions 50 rise to the skin side from rise start portions 59. The rise start portions 59 are portions that extend along the longitudinal direction over the entire length of the rising portions 56. As shown in FIGS. 5, 7B, and the like, the rise start portions 59 are located outward with respect to intersections 121u which is between the lower end of the front waist portion 20 and the side ends 121s of the absorbent core 121 (narrow portion 121n). Also, in the diaper 1, in the region where the front waist portion 20 and the absorbent main body 10 are overlapped with each other, the rise start portions 59 are located outward in the left-right direction with respect to the side ends of the absorbent core 121 (narrow portion 121n).

As shown in the diagram on the left side in FIG. 7B, using the intersection 121u as a reference, due to the rise start portion 59 of the rising portion 56 being located outward in the left-right direction with respect to the intersection 121u, a predetermined space can be provided between the side end of the narrow portion 121n and the rise start portion 59 of the leak-proof wall portion 50 in the left-right direction. If a space is not provided between the side end 121s of the absorbent core 121 and the rise start portion 59, it is not possible to form the leak-prevention space P, or ensure a sufficient size for it, for example. For this reason, the rise start portion 59 is arranged outward in the left-right direction with respect to the intersection 121u, thus making it possible to ensure a sufficient size for the leak-prevention space P.

Next, when the absorbent main body 10 of the diaper 1 has absorbed excrement, the absorbent core 121 expands due to the absorbed excrement or the like, as shown on the right side in FIGS. 7A and 7B. In the present embodiment, the absorbent core 121 contains pulp fibers or the like and SAP, and therefore tends to expand upon absorbing excrement.

As shown on the right side in FIG. 7A, in the left-right-direction cross-section of the absorbent main body 10 taken at the lower ends of the end joining portions 52, the absorbent core 121 has expanded due to absorbing excrement. As shown in FIG. 2 and the like, the leak-proof wall portions 50 are each a sheet member that is continuous in the longitudinal direction (up-down direction), and therefore when one portion of the leak-proof wall portion 50 rises to the skin side, the rising portion 56 rises to the skin side from the rise start portion 59. At this time, in contrast to FIG. 7A, the leak-proof wall portion 50 shown in FIG. 7B is not fixed to the absorbent core 121, and therefore the leak-proof wall portion 50 does not rise directly due to expansion of the absorbent core 121. However, due to the fixed portion 55 fixed to the absorbent core 121, if a portion of the leak-proof wall portion 50 is lifted to the skin side, the rising portion 56 located at the lower end of the front waist portion 20 is lifted to the skin side, and the size of the leak-prevention space can be increased to that of a leak-prevention space Py.

If the leak-proof wall portion 50 is not fixed to the absorbent body unit 12 (absorbent core 121), only the absorbent core 121 and the absorbent body unit 12 that have absorbed excrement rise to the skin side, and the leak-proof wall portion 50 cannot be raised to toward the skin side. Therefore even if the absorbent core 121 expands due to absorbing excrement, the size of the leak-prevention space P cannot be increased.

In the diaper 1, the leak-proof wall portion 50 rises to the skin side, thus making it possible to form the leak-prevention space Py that is larger than the leak-prevention space Px formed before excrement is absorbed, and therefore even if excrement flows quickly toward the front waist portion 20 for example, a larger amount of the excrement can accumulate in the leak-prevention space Py, thus making it possible to suppress the risk of excrement leaking out from the diaper 1.

At this time, the skin-side sheet portion 122a and the non-skin-side sheet portion 122b of the absorbent body unit 12 are adhered to each other, thus making it possible to suppress the expansion of the absorbent core 121 in the left-right direction. Accordingly, the leak-prevention space Py, which is defined by the leak-proof wall portion 50, the absorbent core 121, and the exterior sheet 14, can be formed with a larger size in the left-right direction, thus making it possible to increase the size of the leak-prevention space Py and store a larger amount of excrement.

Furthermore, if the absorbent core 121 absorbs a certain amount of excrement or more and expands, or if the adhesiveness of the adhesive (e.g., hot-melt adhesive) decreases due to moisture, the skin-side sheet portion 122a and the non-skin-side sheet portion 122b may detach from each other. At this time, the absorbent core 121 expands in the left-right direction as well, and the excrement stored in the leak-prevention space P is likely to be more quickly absorbed by the absorbent core 121.

By storing excrement in the leak-prevention space P, it is possible to reduce the risk of the leakage of excrement from the front waist portion 20. Note that excrement stored in the leak-prevention space P can be absorbed by adjacent portions of the absorbent core 121 over time. It should also be noted that because the leak-prevention space P of the diaper 1 is provided in the region that is overlapped with the front waist portion 20 in the front-back direction, if excrement flows to the leak-prevention space P, the absorption of such excrement to adjacent portions of the absorbent core 121 is promoted by compression by the front waist portion 20 and the wearer's body.

Furthermore, it is preferable that portions of the waist elastic members 22 of the diaper 1 are arranged straddling the side ends of the narrow portion 121n in the left-right direction. In the diaper 1, in the lower end portion on the one left-right-direction side of the front waist portion 20, the waist elastic members 22 extend from the left-right-direction end portion of the front waist portion 20 to a position that is inward in the left-right direction with respect to the side end of the narrow portion 121n. Also, at this time, it is more preferable that portions of the waist elastic members 22 intersect the leak-proof-wall elastic members 51. Accordingly, when the diaper 1 is put on, contraction of the waist elastic members 22 can suppress the case where the relatively stiff absorbent main body 10 folds so as to protrude to the non-skin side in the approximately left-right-direction central portion.

Also, as shown in FIG. 5, in the diaper 1 in the stretched state, letting T be the up-down-direction length from the upper recession end 121t of the absorbent core 121 to the lower end of the front waist portion 20, and letting S be the left-right-direction length from the intersection 121u between the lower end of the front waist portion 20 and the side end 121s of the absorbent core 121 to the upper recession end 121t, the length T is greater than the length S (T > S). In other words, the up-down-direction length of the leak-prevention space P is longer than the left-right-direction length thereof. For this reason, excrement that has accumulated in the leak-prevention space P is likely to flow to the region whose left-right-direction length is shorter, due to the capillary phenomenon. Therefore excrement is likely to be stored higher in the up-down direction. This therefore makes it possible to suppress the risk that excrement that has flowed into the leak-prevention space P from the crotch portion returns to the crotch portion.

Furthermore, as shown in FIG. 2, in the diaper 1 in the stretched state, letting the waist elastic members 22 of the front waist portion 20 include a first waist elastic member 22a and a second waist elastic member 22b arranged in this order from the lower side, it is preferable that the length L2 between the first waist elastic member 22a and the lower end of the front waist portion 20 is greater than the length L1 between the first waist elastic member 22a and the second waist elastic member 22b (L1 < L2). According to this configuration, the distance between the lower end of the front waist portion 50 and the first waist elastic member 22a is longer than the distance between the first waist elastic member 22a and the second waist elastic member 22b, and therefore excrement is more likely to enter the leak-prevention space P. On the other hand, due to the first waist elastic member 22a and the second waist elastic member 22b being arranged closer to each other, it is possible to further suppress the risk of outward leakage of excrement that has entered the leak-prevention space P.

As shown in FIG. 2 and the like, in the diaper 1 in the stretched state, it is preferable that portions of the side ends 121s of the absorbent core 121, which are between the lower end of the front waist portion 20 and the upper recession ends 121t of the absorbent core 121, are inclined with respect to the up-down direction. In the diaper 1, the side ends 121s between the upper recession ends 121t and the intersections 121u are inclined so as to extend inward in the left-right direction while extending downward, and therefore are longer than in the case of being parallel with the up-down direction. Accordingly, as shown in FIG. 5 and the like, the surface area of the absorbent core 121 that faces the region corresponding to the leak-prevention space P can be increased, making it possible to increase the size of the region of the absorbent core 121 that comes into contact with excrement in the leak-prevention space P, and making it easier to absorb excrement.

Also, as shown in FIG. 2 and the like, it is preferable that the inner ends of the leak-proof wall portions 50 are inward with respect to the intersections 121u in the left-right direction of the diaper 1 in the stretched state. In other words, it is preferable that the side ends 121s of the absorbent core 121 are covered by the leak-proof wall portions 50 in the region above the intersections 121u. This therefore makes it possible to suppress the risk that excrement absorbed by the absorbent core 121 leaks out from the diaper 1.

Furthermore, it is preferable that the upper end of the narrow portion 121n (i.e., the upper recession ends 121t) is arranged below the lower ends of the end joining portions 52. The upper recession ends 121t are portions that serve as the upper ends of the leak-prevention spaces P (see FIG. 5 and the like). Also, the regions of the leak-proof wall portions 50 that are overlapped with the end joining portions 52 in the up-down direction are the fixed portions 55. Accordingly, if the upper recession ends 121t are arranged above the lower ends of the end joining portions 52 for example, the leak-prevention spaces P become fixed by the fixed portions 55, thus resulting in the risk of a decrease in the size of the leak-prevention spaces P. In view of this, the upper recession ends 121t are arranged below the lower ends of the end joining portions 52, and therefore when the absorbent core 121 expands due to absorbing excrement, the risk that the leak-prevention spaces P become blocked by the expanded absorbent core 121 is reduced, and the leak-prevention spaces P can be increased in size.

Also, as shown in FIG. 2 and the like, it is preferable that the end joining portions 52 of the diaper 1 in the stretched state are arranged inward with respect to the side ends 121s of the absorbent core 121. In the diaper 1, the end joining portions 52 are inward with respect to the side ends 121s in the upper portion of the absorbent core 121, and therefore the leak-prevention spaces P are more likely to be formed when the absorbent core 121 expands due to absorbing excrement.

Furthermore, as shown in FIG. 6, for each of the end joining portions 52 of the diaper 1 in the stretched state, it is preferable that the area of the end joining portion 52 is greater than the area of the fixed portion 55 minus the area of the end joining portion 52 (area of end joining portion 52 > area of fixed portion 55 - area of end joining portion 52). Compared to the case where the area of the end joining portion 52 is smaller than the area of the fixed portion 55 minus the area of the end joining portion 52, the leak-prevention space P is more likely to be formed when the absorbent core 121 expands due to absorbing excrement.

Also, the diaper 1 includes: the front waist portion 20 that has the waist elastic members 22; the back waist portion 30; the absorbent core 121; the skin-side sheet portion 122a; the non-skin-side sheet portion 122b; and the pair of leak-proof wall portions 50 that have the leak-proof-wall elastic members 51. The absorbent core 121 has the narrow portion 121n. The leak-proof wall portions 50 each include the fixed portions 55 and the rising portion 56. The front waist portion 20 is overlapped with the non-skin side of the narrow portion 121n, and furthermore is overlapped with a portion of the narrow portion 121n in the up-down direction. A portion of each of the fixed portions 55 is overlapped with the skin side of the absorbent core 121. Furthermore, letting the intersections 121u between the lower end of the front waist portion 20 and the narrow portion 121n serve as a reference, the rise start portions 59 of the rising portions 56 are arranged outward in the left-right direction with respect to the intersections 121u, and portions of the waist elastic members 22 are arranged straddling the side ends 121s in the left-right direction. Accordingly, with the diaper 1, even when excrement is absorbed by the absorbent core 121 that has the narrow portion 121n in the region where the front waist portion 20 and the absorbent main body 10 are overlapped in the thickness direction, it is possible to suppress the leakage of excrement out from the diaper 1. In addition, this suppresses the risk that the approximately left-right-direction central portion of the relatively stiff absorbent main body 10 folds so as to protrude to the non-skin side, and make it more likely for the absorbent main body 10 to fit to the wearer's body.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof. For example, modification which will be described below is possible.

Although the leak-prevention spaces P are formed on the front waist portion 20 side to suppress the leakage of excrement in the embodiment described above, there is no limitation to this. The leak-prevention spaces P may be formed in the back waist portion 30 as the one-side waist portion, or the leak-prevention spaces P may be formed in both the front waist portion 20 and the back waist portion 30. Forming the leak-prevention spaces P in the back waist portion 30 makes it possible to prevent the leakage of excrement in the back waist portion 30.

Although the waist elastic members 22 are arranged both above and below the upper recession ends 121t in the embodiment described above, there is no limitation to this. A configuration is possible in which, in the region below the upper recession ends 121t in the diaper 1 in the stretched state, the waist elastic members 22 are not arranged in a left-right-direction inward region with respect to the upper recession ends 121t. In other words, a configuration is possible in which the waist elastic members 22 are not arranged in the regions that are overlapped with the leak prevention regions P in the front-back direction. This therefore makes it possible to suppress the risk that the leak-prevention spaces P collapse due to the waist elastic members 22, and that excrement cannot be stored in the leak-prevention spaces P.

### [Reference Signs List]

1 diaper (pull-on disposable diaper, absorbent article),
10 absorbent main body,
10f front upper end portion, 10b back upper end portion,
11 top sheet,
12 absorbent body unit,
121 absorbent core,
121n narrow portion, 121s side end,
121t upper recession end, 121u intersection,
122 core-wrapping sheet,
122a skin-side sheet portion, 122b non-skin-side sheet portion,
122Ha skin-side adhesive portion, 122Hb non-skin side adhesive portion,
13 back sheet, 14 exterior sheet,
20 front waist portion (one-side waist portion), 20a side portion,
21 sheet, 22 waist elastic member,
30 back waist portion, 30a side portion,
31 sheet, 32 waist elastic member,
50 leak-proof wall portion (leak-proof wall), 51 leak-proof-wall elastic member,
52 end joining portion (adhesion portion), 53 side joining portion,
55 fixed portion, 56 rising portion,
59 rise start portion,
60 leg portion, 61 leg elastic member,
LH leg opening, BH waist opening,
P leak-prevention space

## Claims

1. An absorbent article (1) having an up-down direction, a left-right direction, and a front-back direction that intersect each other,
the absorbent article comprising:
an absorbent main body (10) including an absorbent body (121), a core-wrapping sheet (122) having a skin-side sheet portion (122a) and a non-skin-side sheet portion (122b), and a pair of leak-proof walls,
the absorbent body (121) having a superabsorbent polymer,
the skin-side sheet portion (122a) located on a skin side of the absorbent body (121),
the non-skin-side sheet portion (122b) located on a non-skin side of the absorbent body (121),
the pair of leak-proof walls (50) each having a leak-proof-wall elastic member (51);
a front waist portion (20); and
a back waist portion (30),
the absorbent body (121) including a narrow portion (121n) that has a left-right-direction side end (121s) having a recessed portion,
the leak-proof walls each including a fixed portion (55) and a rising portion (56),
the fixed portion (55) being not capable of rising to the skin side,
the rising portion (56) being capable of rising to the skin side, out of the front waist portion (20) and the back waist portion (30) ,
at least one-side waist portion that is located on one side being overlapped with a non-skin side of the narrow portion (121n),
the at least one-side waist portion being overlapped in the up-down direction with at least a portion of the narrow portion (121n),
an adhesive (122Ha, 122Hb) being applied to at least either one of a non-skin-side surface of the skin-side sheet portion (122a) and a skin-side surface of the non-skin-side sheet portion (122b),
the application being made in a region that is at least outward of the absorbent core which includes the narrow portion (121n)above a lower end of the one-side waist portion,
at least a portion of the fixed portion (55) being overlapped with the skin side of the absorbent body (121),
when an intersection (121u) between the lower end of the one-side waist portion and the side end (121s) of the narrow portion (121n) is used as a reference,
a rise start portion (59) of the rising portion (56) being located outward in the left-right direction with respect to the intersection (121u), wherein
in a stretched state where the leak-proof-wall elastic member (51) is stretched,
concerning an upper recession end (121t) that is an upper end of a located-on-the-one-side and recessed portion of the side end (121s),
an up-down-direction length (T) from the upper recession end (121t) to the lower end of the one-side waist portion
is longer than
a left-right-direction length (S) from the upper recession end (121t) to an intersection (121u) between the lower end of the one-side waist portion and the side ends (121s).

2. The absorbent article according to claim 1, wherein
the one-side waist portion has a waist elastic member (22) that extends in the left-right direction, and
at least a portion of the waist elastic member (22) straddles the side end (121s) of the narrow portion (121n) in the left-right direction.

3. The absorbent article according to claim 2, wherein
at least a portion of the waist elastic member (22) intersects the leak-proof-wall elastic member (51).

4. The absorbent article according to claim 1, wherein
the one-side waist portion has a waist elastic member (22) that extends in the left-right direction, and
in a stretched state where the waist elastic member (22) and the leak-proof-wall elastic member (51) are stretched,
concerning an upper recession end (121t) that is an upper end of a located-on-the-one-side and recessed portion of the side end (121s),
the waist elastic member (22) that is located below the upper recession end (121t) is not provided inward in the left-right direction with respect to the upper recession ends when the upper recession end (121t) is used as a reference.

5. The absorbent article according to any one of claims 1 to 4, wherein
in a stretched state where the leak-proof-wall elastic member (51) is stretched,
concerning an upper recession end (121t) that is an upper end of a located-on-the-one-side and recessed portion of the side end (121s),
a portion of the side end (121s) is inclined with respect to the up-down direction, the portion of the side end (121s) being located between the upper recession end (121t) and the lower end of the one-side waist portion.

6. The absorbent article according to any one of claims 1 to 5, wherein
on the one side, an upper end of the narrow portion (121n) is located below a lower end of the fixed portion (55).

7. The absorbent article according to any one of claims 1 to 6, wherein
in a stretched state where the leak-proof-wall elastic member (51) is stretched,
an inner end of each of the leak-proof wall (50) is located inward in the left-right direction with respect to the intersection (121u).

8. The absorbent article according to any one of claims 1 to 7, wherein
in a stretched state where the leak-proof-wall elastic member (51) is stretched,
the leak-proof walls (50) each have an adhesion portion (52) for preventing rising of the fixed portion (55) to the skin side, and
the adhesion portion (52) is located inward with respect to the side ends (121s) of the absorbent body (121).

9. The absorbent article according to any one of claims 1 to 8, wherein
in a stretched state where the leak-proof-wall elastic member (51) is stretched,
the leak-proof walls (50) each have an adhesion portion (52) for preventing rising of the fixed portion (55) to the skin side, and
the area of the adhesion portions (52) is greater than the area of the fixed portions (55) minus the area of the adhesion portions (52) .

10. The absorbent article according to any one of claims 1 to 9, wherein
the one-side waist portion has a plurality of waist elastic members (22) that extend in the left-right direction, and
in a stretched state where the waist elastic members (22) and the leak-proof-wall elastic member (51) are stretched,
concerning a first waist elastic member (22a) that is one of the plurality of waist elastic members and that is located lowest in the one-side waist portion, and
concerning a second waist elastic member (22b) that is one of the plurality of waist elastic members and that is vertically adjacent to the first waist elastic member (22a),
a length (L2) from the lower end of the one-side waist portion to the first waist elastic member (22a) is longer than a length (L1) between the first waist elastic member (22a) and the second waist elastic member (22b) .

11. The absorbent article according to any one of claims 1 to 10, wherein
in a stretched state where the leak-proof-wall elastic member (51) is stretched,
the leak-proof-wall elastic member (51) includes a first leak-proof-wall elastic member and a second leak-proof-wall elastic member in the rising portions (56), and
the first leak-proof-wall elastic member and the second leak-proof-wall elastic member are separated from each other.

12. The absorbent article according to any one of claims 1 to 11, wherein adhesive portions (122Ha, 122Hb) are provided at least outward of the absorbent core (121) which includes the narrow portion (121n) , in a region upward of the lower end of the one-side waist portion.

## Patentansprüche

1. Absorbierender Artikel (1), der eine Oben-Unten-Richtung, eine Links-Rechts-Richtung und eine Vorn-Hinten-Richtung aufweist, die einander schneiden,
wobei der absorbierende Artikel Folgendes umfasst:
einen absorbierenden Hauptkörper (10), der Folgendes einschließt: einen Saugkörper (121), eine Kernumhüllungslage (122), die einen Hautseitenlagenteil (122a) und einen Nicht-Hautseitenlagenteil (122b) aufweist, und ein Paar von auslaufsicheren Wänden,
wobei der Saugkörper (121) ein superabsorbierendes Polymer aufweist,
sich der Hautseitenlagenteil (122a) auf einer Hautseite des Saugkörpers (121) befindet,
sich der Nicht-Hautseitenlagenteil (122b) auf einer Nicht-Hautseite des Saugkörpers (121) befindet,
das Paar von auslaufsicheren Wänden (50) jeweils ein elastisches Element der auslaufsicheren Wand (51) aufweist;
einen vorderen Taillenteil (20); und
einen hinteren Taillenteil (30),
wobei der Saugkörper (121) einen schmalen Teil (121n) einschließt, der ein Seitenende in Links-Rechts-Richtung (121s) aufweist, das einen ausgesparten Teil aufweist,
die auslaufsicheren Wände jeweils einen fixierten Teil (55) und einen aufstehenden Teil (56) einschließen,
wobei der fixierte Teil (55) nicht zu der Hautseite aufstehen kann,
der aufstehende Teil (56) zu der Hautseite aufstehen kann, aus dem vorderen Taillenteil (20) und dem hinteren Taillenteil (30),
ein mindestens einseitiger Taillenteil, der sich auf einer Seite befindet, mit einer Nicht-Hautseite des schmalen Teils (121n) überlappt ist,
der mindestens einseitige Taillenteil in der Oben-Unten-Richtung mit mindestens einem Teil des schmalen Teils (121n) überlappt ist,
ein Haftmittel (122Ha, 122Hb), das auf mindestens eine von einer Nicht-Hautseitenoberfläche des Hautseitenlagenteils (122a) und einer Hautseitenoberfläche des Nicht-Hautseitenlagenteils (122b) aufgetragen ist,
wobei die Auftragung in einem Bereich durchgeführt ist, der sich zumindest äußerlich des Saugkerns befindet, der den schmalen Teil (121n) über einem unteren Ende des einseitigen Taillenteils einschließt,
mindestens ein Teil des fixierten Teils (55) mit der Hautseite des Saugkörpers (121) überlappt ist,
wenn eine Schnittstelle (121u) zwischen dem unteren Ende des einseitigen Taillenteils und dem Seitenende (121s) des schmalen Teils (121n) als Bezug verwendet wird,
sich ein Teil des aufstehenden Beginns (59) des aufstehenden Teils (56) äußerlich in der Links-Rechts-Richtung in Bezug auf die Schnittstelle (121u) befindet, wobei
in einem gedehnten Zustand, wo das elastisches Element der auslaufsicheren Wand (51) gedehnt ist,
hinsichtlich eines oberen Aussparungsendes (121t), das ein oberes Ende eines sich auf der einen Seite befindlichen und ausgesparten Teils des Seitenendes (121s) ist,
eine Länge in der Oben-Unten-Richtung (T) von dem oberen Aussparungsende (121t) zu dem unteren Ende des einseitigen Taillenteils
länger ist als
eine Länge in der Links-Rechts-Richtung (S) von dem oberen Aussparungsende (121t) zu einem Schnittpunkt (121u) zwischen dem unteren Ende des einseitigen Taillenteils und den Seitenenden (121s).

2. Absorbierender Artikel nach Anspruch 1, wobei
der einseitige Taillenteil ein elastisches Taillenelement (22) aufweist, das sich in der Links-Rechts-Richtung erstreckt, und
mindestens ein Teil des elastischen Taillenelements (22) das Seitenende (121s) des schmalen Teils (121n) in der Links-Rechts-Richtung überspannt.

3. Absorbierender Artikel nach Anspruch 2, wobei
mindestens ein Teil des elastischen Taillenelements (22) das elastisches Element der auslaufsicheren Wand (51) schneidet.

4. Absorbierender Artikel nach Anspruch 1, wobei
der einseitige Taillenteil ein elastisches Taillenelement (22) aufweist, das sich in der Links-Rechts-Richtung erstreckt, und
in einem gedehnten Zustand, wo das elastische Taillenelement (22) und das elastische Element der auslaufsicheren Wand (51) gedehnt sind,
hinsichtlich eines oberen Aussparungsendes (121t), das ein oberes Ende eines sich auf der einen Seite befindlichen und ausgesparten Teils des Seitenendes (121s) ist,
das elastische Taillenelement (22), das sich unter dem oberen Aussparungsende (121t) befindet, nicht nach innen in der Links-Rechts-Richtung in Bezug auf die oberen Aussparungsenden bereitgestellt ist, wenn das obere Aussparungsende (121t) als Bezug verwendet wird.

5. Absorbierender Artikel nach einem der Ansprüche 1 bis 4, wobei
in einem gedehnten Zustand, wo das elastische Element der auslaufsicheren Wand (51) gedehnt ist,
hinsichtlich eines oberen Aussparungsendes (121t), das ein oberes Ende eines sich auf der einen Seite befindlichen und ausgesparten Teils des Seitenendes (121s) ist,
ein Teil des Seitenendes (121s) in Bezug auf die Oben-Unten-Richtung geneigt ist, wobei sich der Teil des Seitenendes (121s) zwischen dem oberen Aussparungsende (121t) und dem unteren Ende des einseitigen Taillenteils befindet.

6. Absorbierender Artikel nach einem der Ansprüche 1 bis 5, wobei
sich auf der einen Seite ein oberes Ende des schmalen Teils (121n) unter einem unteren Ende des fixierten Teils (55) befindet.

7. Absorbierender Artikel nach einem der Ansprüche 1 bis 6, wobei
in einem gedehnten Zustand, wo das elastische Element der auslaufsicheren Wand (51) gedehnt ist,
sich ein inneres Ende von jeder der auslaufsicheren Wände (50) nach innen in der Links-Rechts-Richtung in Bezug auf die Schnittstelle (121u) befindet.

8. Absorbierender Artikel nach einem der Ansprüche 1 bis 7, wobei
in einem gedehnten Zustand, wo das elastische Element der auslaufsicheren Wand (51) gedehnt ist,
die auslaufsicheren Wände (50) jeweils einen Haftungsteil (52) zur Verhinderung des Aufstehens des fixierten Teils (55) zu der Hautseite aufweisen und
sich der Haftungsteil (52) nach innen in Bezug auf die Seitenenden (121s) des Saugkörpers (121) befindet.

9. Absorbierender Artikel nach einem der Ansprüche 1 bis 8, wobei
in einem gedehnten Zustand, wo das elastische Element der auslaufsicheren Wand (51) gedehnt ist,
die auslaufsicheren Wände (50) jeweils einen Haftungsteil (52) zur Verhinderung des Aufstehens des fixierten Teils (55) zu der Hautseite aufweisen und
die Fläche der Haftungsteile (52) größer ist als die Fläche der fixierten Teile (55) abzüglich der Fläche der Haftungsteile (52).

10. Absorbierender Artikel nach einem der Ansprüche 1 bis 9, wobei
der einseitige Taillenteil eine Vielzahl von elastischen Taillenelementen (22) aufweist, die sich in der Links-Rechts-Richtung erstrecken, und
in einem gedehnten Zustand, wo das elastische Taillenelement (22) und das elastische Element der auslaufsicheren Wand (51) gedehnt sind,
hinsichtlich eines ersten elastischen Taillenelements (22a), das eines der Vielzahl von elastischen Taillenelementen ist und das sich am untersten in dem einseitigen Taillenteil befindet, und
hinsichtlich eines zweiten elastischen Taillenelements (22b), das eines der Vielzahl von elastischen Taillenelementen ist und das vertikal benachbart zu dem ersten elastischen Taillenelement (22a) vorliegt,
eine Länge (L2) von dem unteren Ende des einseitigen Taillenteils zu dem ersten elastischen Taillenelement (22a) länger ist als eine Länge (L1) zwischen dem ersten elastischen Taillenelement (22a) und dem zweiten elastischen Taillenelement (22b).

11. Absorbierender Artikel nach einem der Ansprüche 1 bis 10, wobei
in einem gedehnten Zustand, wo das elastische Element der auslaufsicheren Wand (51) gedehnt ist,
das elastische Element der auslaufsicheren Wand (51) ein erstes elastisches Element der auslaufsicheren Wand und ein zweites elastisches Element der auslaufsicheren Wand in den aufstehenden Teilen (56) einschließt und
das erste elastische Element der auslaufsicheren Wand und das zweite elastische Element der auslaufsicheren Wand voneinander getrennt sind.

12. Absorbierender Artikel nach einem der Ansprüche 1 bis 11, wobei Haftmittelteile (122Ha, 122Hb) zumindest äußerlich des Saugkerns (121), der den schmalen Teil (121n) einschließt, in einem Bereich nach oben von dem unteren Ende des einseitigen Taillenteils bereitgestellt sind.

## Revendications

1. Article absorbant (1) ayant une direction allant de haut en bas, une direction allant de gauche à droite, et une direction allant d'avant en arrière qui se croisent les unes les autres,
l'article absorbant comportant :
un corps principal absorbant (10) comprenant un corps absorbant (121), une feuille enveloppant la partie centrale (122) ayant une partie de feuille côté orienté vers la peau (122a) et une partie de feuille côté non orienté vers la peau (122b), et une paire de parois antifuites,
le corps absorbant (121) ayant un polymère superabsorbant,
la partie de feuille côté orienté vers la peau (122a) étant située sur un côté orienté vers la peau du corps absorbant (121),
la partie de feuille côté non orienté vers la peau (122b) étant située sur un côté non orienté vers la peau du corps absorbant (121),
les parois de la paire de parois antifuites (50) ayant, chacune, un élément élastique au niveau de la paroi antifuite (51) ;
une partie avant au niveau de la taille (20) ; et
une partie arrière au niveau de la taille (30),
le corps absorbant (121) comprenant une partie étroite (121n) qui a une extrémité latérale dans la direction allant de gauche à droite (121s) ayant une partie en retrait,
les parois antifuites comprenant, chacune, une partie fixe (55) et une partie en surélévation (56),
la partie fixe (55) n'étant pas en mesure de se surélever sur le côté orienté vers la peau,
la partie en surélévation (56) étant en mesure de se surélever sur le côté orienté vers la peau, hors de la partie avant au niveau de la taille (20) et de la partie arrière au niveau de la taille (30),
au moins une partie d'un côté au niveau de la taille se trouvant sur un côté étant chevauchée par un côté non orienté vers la peau de la partie étroite (121n),
ladite au moins une partie d'un côté au niveau de la taille étant chevauchée dans la direction allant de haut en bas par au moins une partie de la partie étroite (121n),
un adhésif (122Ha, 122Hb) étant appliqué sur au moins l'une ou l'autre parmi une surface de côté non orienté vers la peau de la partie de feuille côté orienté vers la peau (122a) et une surface de côté orienté vers la peau de la partie de feuille côté non orienté vers la peau (122b),
l'application étant effectuée dans une région qui est au moins dirigée vers l'extérieur depuis la partie centrale absorbante qui comprend la partie étroite (121n) au-dessus d'une extrémité inférieure de la partie d'un côté au niveau de la taille,
au moins une partie de la partie fixe (55) étant chevauchée par le côté orienté vers la peau du corps absorbant (121),
quand une intersection (121u) entre l'extrémité inférieure de la partie d'un côté au niveau de la taille et l'extrémité latérale (121s) de la partie étroite (121n) est utilisée pour servir de référence,
une partie de départ de surélévation (59) de la partie en surélévation (56) étant située vers l'extérieur dans la direction allant de gauche à droite par rapport à l'intersection (121u), dans lequel
dans un état étiré dans lequel l'élément élastique au niveau de la paroi antifuite (51) est étiré,
concernant une extrémité de mise en retrait supérieure (121t) qui est une extrémité supérieure d'une partie située sur ledit un côté et en retrait de l'extrémité latérale (121s),
une longueur dans la direction allant de haut en bas (T) depuis l'extrémité de mise en retrait supérieure (121t) jusqu'à l'extrémité inférieure de la partie d'un côté au niveau de la taille
est plus longue par rapport à
une longueur dans la direction allant de gauche à droite (S) depuis l'extrémité de mise en retrait supérieure (121t) jusqu'à une intersection (121u) entre l'extrémité inférieure de la partie d'un côté au niveau de la taille et les extrémités latérales (121s).

2. Article absorbant selon la revendication 1, dans lequel
la partie d'un côté au niveau de la taille a un élément élastique au niveau de la taille (22) qui s'étend dans la direction allant de gauche à droite, et
au moins une partie de l'élément élastique au niveau de la taille (22) enjambe l'extrémité latérale (121s) de la partie étroite (121n) dans la direction allant de gauche à droite.

3. Article absorbant selon la revendication 2, dans lequel
au moins une partie de l'élément élastique au niveau de la taille (22) croise l'élément élastique au niveau de la paroi antifuite (51).

4. Article absorbant selon la revendication 1, dans lequel
la partie d'un côté au niveau de la taille a un élément élastique au niveau de la taille (22) qui s'étend dans la direction allant de gauche à droite, et
dans un état étiré dans lequel l'élément élastique au niveau de la taille (22) et l'élément élastique au niveau de la paroi antifuite (51) sont étirés,
concernant une extrémité de mise en retrait supérieure (121t) qui est une extrémité supérieure de la partie située sur ledit un côté et en retrait de l'extrémité latérale (121s),
l'élément élastique au niveau de la taille (22) qui est situé sous l'extrémité de mise en retrait supérieure (121t) n'est pas mis en œuvre vers l'intérieur dans la direction allant de gauche à droite par rapport aux extrémités de mise en retrait supérieures quand l'extrémité de mise en retrait supérieure (121t) est utilisée pour servir de référence.

5. Article absorbant selon l'une quelconque des revendications 1 à 4, dans lequel
dans un état étiré dans lequel l'élément élastique au niveau de la paroi antifuite (51) est étiré,
concernant une extrémité de mise en retrait supérieure (121t) qui est une extrémité supérieure d'une partie située sur ledit un côté et en retrait de l'extrémité latérale (121s),
une partie de l'extrémité latérale (121s) est inclinée par rapport à la direction allant de haut en bas, la partie de l'extrémité latérale (121s) étant située entre l'extrémité de mise en retrait supérieure (121t) et l'extrémité inférieure de la partie d'un côté au niveau de la taille.

6. Article absorbant selon l'une quelconque des revendications 1 à 5, dans lequel
sur ledit un côté, une extrémité supérieure de la partie étroite (121n) est située sous une extrémité inférieure de la partie fixe (55).

7. Article absorbant selon l'une quelconque des revendications 1 à 6, dans lequel
dans un état étiré dans lequel l'élément élastique au niveau de la paroi antifuite (51) est étiré,
une extrémité intérieure de chacune des parois antifuites (50) est située vers l'intérieur dans la direction allant de gauche à droite par rapport à l'intersection (121u).

8. Article absorbant selon l'une quelconque des revendications 1 à 7, dans lequel
dans un état étiré dans lequel l'élément élastique au niveau de la paroi antifuite (51) est étiré,
les parois antifuites (50) ont, chacune, une partie d'adhésion (52) servant à empêcher toute surélévation de la partie fixe (55) sur le côté orienté vers la peau, et
la partie d'adhésion (52) est située vers l'intérieur par rapport aux extrémités latérales (121s) du corps absorbant (121) .

9. Article absorbant selon l'une quelconque des revendications 1 à 8, dans lequel
dans un état étiré dans lequel l'élément élastique au niveau de la paroi antifuite (51) est étiré,
les parois antifuites (50) ont, chacune, une partie d'adhésion (52) servant à empêcher toute surélévation de la partie fixe (55) sur le côté orienté vers la peau, et
la surface des parties d'adhésion (52) est supérieure à la surface des parties fixes (55) moins la surface des parties d'adhésion (52).

10. Article absorbant selon l'une quelconque des revendications 1 à 9, dans lequel
la partie d'un côté au niveau de la taille a une pluralité d'éléments élastiques au niveau de la taille (22) qui s'étendent dans la direction allant de gauche à droite, et
dans un état étiré dans lequel les éléments élastiques au niveau de la taille (22) et l'élément élastique au niveau de la paroi antifuite (51) sont étirés,
concernant un premier élément élastique au niveau de la taille (22a) qui et l'un de la pluralité d'éléments élastiques au niveau de la taille et qui est situé au plus bas dans la partie d'un côté au niveau de la taille, et
concernant un deuxième élément élastique au niveau de la taille (22b) qui est l'un de la pluralité d'éléments élastiques au niveau de la taille et qui est adjacent à la verticale par rapport au premier élément élastique au niveau de la taille (22a),
une longueur (L2) depuis l'extrémité inférieure de la partie d'un côté au niveau de la taille jusqu'au premier élément élastique au niveau de la taille (22a) est plus longue par rapport à une longueur (L1) entre le premier élément élastique au niveau de la taille (22a) et le deuxième élément élastique au niveau de la taille (22b).

11. Article absorbant selon l'une quelconque des revendications 1 à 10, dans lequel
dans un état étiré dans lequel l'élément élastique au niveau de la paroi antifuite (51) est étiré,
l'élément élastique au niveau de la paroi antifuite (51) comprend un élément élastique au niveau de la paroi antifuite et un deuxième élément élastique au niveau de la paroi antifuite dans les parties en surélévation (56), et
le premier élément élastique au niveau de la paroi antifuite et le deuxième élément élastique au niveau de la paroi antifuite sont séparés l'un de l'autre.

12. Article absorbant selon l'une quelconque des revendications 1 à 11, dans lequel des parties adhésives (122Ha, 122Hb) sont mises en œuvre au moins vers l'extérieur depuis la partie centrale absorbante (121) qui comprend la partie étroite (121n), dans une région vers le haut depuis l'extrémité inférieure de la partie d'un côté au niveau de la taille.
